# EUROPÄISCHE PATENTANMELDUNG

(11) **EP 4 437 893 A1**
(43) Veröffentlichungstag der Anmeldung: **02.10.2024**
(21) Anmeldenummer: 24151121.1
(22) Anmeldetag: 10.01.2024
(51) Int. Cl.: A41D 31/26, D06C 29/00, A61N 1/16, A44C 5/00

(54) **KÖRPERNAH ZU TRAGENDES ERZEUGNIS SOWIE VERFAHREN ZUR HERSTELLUNG EINER IN KÖRPERNAH ZU TRAGENDE ERZEUGNISSE EINZUBRINGENDEN, ENERGETISCH MODIFIZIERTEN TRÄGERSUBSTANZ**

(30) Priorität: 11.01.2023 DE 202023100108 U
(71) Anmelder: Penergetic International AG, 8590 Romanshorn (CH)
(72) Erfinder: Plocher, Daniel, 8590 Romanshorn (CH)
(74) Vertreter: Werner, André

(57) **Zusammenfassung**

Die Erfindung betrifft ein Bekleidungsstück, das zum allgemeinen Wohlbefinden beim Träger oder Benutzer des Erzeugnisses beitragen soll, sowie ein Verfahren zur Herstellung einer in einem Bekleidungsstück einzubringenden, energetisch modifizierten Trägersubstanz. Die in das Bekleidungsstück eingebrachte bzw. einzubringende energetisch modifizierte Trägersubstanz ist aus einer primären Trägersubstanz mit einem definierten elektromagnetischen Primäreigenschaftsspektrum gebildet, dem zur Ausbildung eines Zieleigenschaftsspektrums ein elektromagnetisches Prägeeigenschaftsspektrum einer Prägesubstanz aufgeprägt ist bzw. wird.

## Beschreibung

Die Erfindung betrifft ein körpernah zu tragendes Erzeugnis, das zum allgemeinen Wohlbefinden beim Träger oder Benutzer des Erzeugnisses beitragen soll, sowie ein Verfahren zur Herstellung einer in körpernah zu tragende Erzeugnisse einzubringenden, energetisch modifizierten Trägersubstanz.

Es ist allgemein bekannt, dass Organismen mit magnetischen Feldern in Wechselwirkung treten bzw. auf magnetische Felder reagieren. So ist zum Beispiel die Fähigkeit, das Erdmagnetfeld wahrzunehmen und sich an ihm zu orientieren, im Tierreich weit verbreitet.

Im Allgemeinen werden elektrische und magnetische Felder von Lebewesen aber nur latent erfasst. Die Nervenaktivität, zum Beispiel, die auf elektrische Ströme beruht, ist mittels äußerer elektrischer oder magnetischer Felder wahrnehmbar oder beinflussbar, da die elektrischen Ströme in Nervenbahnen und die diese elektrischen Ströme begleitenden Magnetfelder mit den äußeren Feldern elektromagnetisch interagieren können.

Das weitaus stärkste auf die Organismen wirkende Magnetfeld auf der Erde ist das Erdmagnetfeld, welches makroskopisch nahezu alle anderen magnetischen Felder überdeckt. Allerdings können auch kleinere magnetische Felder - ebenso wie elektrische Felder - auf biologische Abläufe in Organismen einwirken. Dies ist vor allem dann der Fall, wenn die Quelle des meist in seiner Wirkweite räumlich begrenzten elektrischen oder magnetischen Feldes in die Nähe oder in Kontakt von Organismen gebracht wird.

Da alle Stoffe eine auf ihre Elementarteilchen zurückgehende Form des Magnetismus besitzen, wirkt jeder Stoff bei Kontakt mit einem Organismus - neben der stofflichen Interaktion - auch durch die dem Stoff eigenen magnetischen als auch elektrischen Felder. Für Wechselwirkungen bedarf es, da magnetischen Feldern eine gewisse Fernwirkung zu eigen ist, nicht zwingend eines unmittelbaren stofflichen Kontaktes bzw. eventueller chemischer Reaktionen.

Die von Stoffen ausgehenden magnetischen Felder gehen auf einer Vielzahl von magnetischen Momenten der im Stoff enthaltenden Atome und deren Elementarteilchen zurück. Die magnetischen Momente der Elementarteilchen sind mit ihrem Spin verknüpft. Abgesehen von ferromagnetischen Stoffen neutralisieren sich bei den meisten Stoffen die magnetischen Momente von Atomen und Elementarteilchen, sodass makroskopisch im Regelfall nur eine geringe, aber vorhandene Wirkung verbleibt. Zudem sind die stoffeigenen Magnetfelder gegenüber dem Erdmagnetfeld in der Regel so gering, dass sie nur mit hohem Aufwand erfassbar sind.

Nichtsdestotrotz besitzt jeder Stoff ein auf den magnetischen Momenten beruhendes, für diesen Stoff spezifisches elektromagnetisches Eigenschaftsspektrum bzw. elektromagnetisches Charakteristikum, welches ähnlich individuell für diesen Stoff ist, wie zum Beispiel die ebenfalls durch den Elektromagnetismus hervorgerufene Farbgebung. Insbesondere organische Substanzen, vor allem die aus biologischen Prozessen stammenden Substanzen, zeichnen sich durch ein komplexes, auf den magnetischen Momenten der Atome und Elementarteilchen beruhendes, arteigenes elektromagnetisches Eigenschaftsspektrum aus. In anschaulicher Form kann dies durch ein stoffspezifisches Schwingungsmuster mit einer oder mehreren spezifischen Frequenzen bzw. einem spezifischen Frequenzmuster charakterisiert werden. Diese Schwingungen haben - bedingt durch die geringe Reichweite der Magnetfelder der magnetischen Momente der Atome und Elementarteilchen - eine begrenzte Reichweite und sind mithin vor allem in räumlicher Nähe zur Substanz wirksam.

So wie eine Vielzahl von Substanzen durch ihre stoffliche Zusammensetzung bei chemischer Wechselwirkung physiologisch wirksam sein kann, können auch die auf magnetischen Momenten basierenden elektromagnetischen Stoffeigenschaften physiologisch wirksam werden, wenngleich es mitunter schwierig ist, diese elektromagnetischen Wechselwirkungen von den chemischen Wechselwirkungen zu separieren.

Es ist ferner unbestritten, dass bestimmte Substanzen für das allgemeine Wohlbefinden vorteilhaft wirken können bzw. physiologisch wirksam sind, auch wenn deren genaue Wirkungsweise nicht oder gegebenenfalls noch nicht in allen Einzelheiten erforscht ist. So ist es unter anderem möglich, durch schlichten Kontakt mit Körpern oder Substanzen physiologisch positive Effekte zu erzielen. Diese Effekte werden unter anderem bei Naturheilverfahren und den sanften Heilverfahren genutzt. Zu den Mechanismen, die bei solchen Anwendungen wirksam werden, sind auch die durch Elektromagnetismus ausgelösten physiologischen Effekte zu zählen.

Da eine Reihe von physiologisch positiv bzw. vorteilhaft wirkenden Substanzen häufig nicht in genügender Menge zur Verfügung stehen, ist es wünschenswert, zumindest das auf den magnetischen Momenten basierende elektromagnetische Eigenschaftsspektrum in anderen Stoffen oder Substanzen durch deren energetische Modifizierung nutzbar zu machen.

Aufgabe der Erfindung ist es, ein energetisch modifiziertes, körpernah zu tragendes Erzeugnis bereitzustellen, welches zumindest in Teilen die elektromagnetischen Eigenschaften einer spezifizierten, vorzugsweise einer physiologisch vorteilhaft wirkenden Substanz aufweist.

Diese Aufgabe wird durch ein körpernah zu tragendes Erzeugnis mit den Merkmalen nach Anspruch 1 sowie durch ein Verfahren zur Herstellung einer in körpernah zu tragende Erzeugnisse einzubringenden, energetisch modifizierten Trägersubstanz nach Anspruch 14 gelöst; zweckmäßige Weiterbildungen der Erfindung sind in den Ansprüchen 2 bis 13 sowie 15 beschrieben.

Nach Maßgabe der Erfindung weist das körpernah zu tragende Erzeugnis mindestens eine energetisch modifizierte Trägersubstanz mit einem durch die magnetischen Momente der Atome und Elementarteilchen definierten elektromagnetischen Zieleigenschaftsspektrum auf.

Die energetisch modifizierte Trägersubstanz ist aus einer primären Trägersubstanz durch energetische Modifikation auf der Ebene der Atome und Elementarteilchen gebildet. Die primäre Trägersubstanz besitzt ein durch die magnetischen Momente der Atome und Elementarteilchen der primären Trägersubstanz definiertes elektromagnetisches Primäreigenschaftsspektrum. Diesem elektromagnetischen Primäreigenschaftsspektrum ist durch die energetische Modifikation ein elektromagnetisches Prägespektrum aufgeprägt bzw. aufmoduliert. Das elektromagnetische Prägespektrum wiederum ist durch die magnetischen Momente der Atome und Elementarteilchen einer Prägesubstanz definiert.

Erhältlich ist die energetisch modifizierte Trägersubstanz insbesondere, indem zunächst das elektromagnetische Prägeeigenschaftsspektrum der Prägesubstanz in Form eines Frequenzmusters erfasst und anschließend dieses erfasste Frequenzmuster auf die Trägersubstanz durch elektromagnetisch induzierte Modifikation der magnetischen Momente der Atome und Elementarteilchen der primären Trägersubstanz übertragen wird, wobei sich die energetisch modifizierte Trägersubstanz mit dem elektromagnetischen Zieleigenschaftsspektrum ausbildet.

Die energetische Modifikation der Trägersubstanz kann zum Beispiel durch Manipulation des Spins - und damit der magnetischen Momente - der elementaren Grundbausteine der Trägersubstanz mittels magnetischer und elektrischer Felder durchgeführt werden.

Die energetisch modifizierte Trägersubstanz umfasst nach der Modifikation einen Teil der elektromagnetischen Eigenschaften der Prägesubstanz, und zwar in der Form, dass diese der Trägersubstanz elektromagnetisch aufgeprägt sind; oder anders ausgedrückt: die Trägersubstanz ist mit den elektromagnetischen Eigenschaften der Prägesubstanz energetisch programmiert.

Die energetisch modifizierte Trägersubstanz weist infolge des Energieeintrags durch die energetische Modifikation gegenüber der primären Trägersubstanz einen höheren Energiegehalt auf. Aufgrund dieses größeren Energiegehaltes treten bei bestimmungsgemäßem Gebrauch, d. h. bei körpernahem Tragen des Erzeugnisses, verstärkt Wechselwirkungen der energetisch modifizierten Trägersubstanz mit der unmittelbaren Umgebung, also zum Beispiel mit der Haut bei einem auf der Haut getragenem Erzeugnis, auf. Die Wechselwirkung mit der Umgebung erfolgt insbesondere in Form des zuvor übertragenen Frequenzmusters. Die Atome und Elementarteilchen der Körperteile, an denen das Erzeugnis getragen wird, werden energetisch angeregt; es können zum Beispiel Resonanzen ausgelöst werden.

Durch die körpernahe Anwendung ist sichergestellt, dass die elektromagnetischen Wirkungen durch die magnetischen Momente der Atome und Elementarteilchen wirksam werden können, auch wenn die stoffgebundenen elektrischen oder magnetischen Felder nur eine begrenzte Ausdehnung aufweisen. In Kontaktbereichen bzw. in kontaktnahen Bereichen können magnetische Momente, zum Beispiel in einzelnen Molekülen, besonders wirksam die Umgebung beeinflussen.

Da die Wechselwirkungen mit biologischen Systemen bzw. mit Teilen biologischer Systeme, zu denen auch die vom Erzeugnis berührten bzw. kontaktierten Körperteile gehören, vor allem mit der aufgeprägten elektromagnetischen Charakteristik der Prägesubstanz erfolgen, wirkt die energetisch modifizierte Trägersubstanz physiologisch in ähnlicher oder vergleichbare Weise wie die Prägesubstanz. In gewissem Umfang besitzt die energetisch modifizierte Trägersubstanz somit einen Teil der Wirkkomplexität der Prägesubstanz.

Als Prägesubstanzen eignen sich vor allem physiologisch positiv bzw. vorteilhaft wirkende Substanzen, deren elektromagnetische, d. h. durch das elektromagnetische Prägeeigenschaftsspektrum bestimmte, Wirkungen auf die Trägersubstanz übertragbar sind. Somit können die körpernah zu tragenden Erzeugnisse bei bestimmungsgemäßer Anwendung aufgrund ihrer energetisch modifizierten Trägersubstanz im gewissen Umfang ähnlich physiologisch vorteilhaft wie die Prägesubstanzen wirken.

Gemäß einer Ausgestaltung der Erfindung ist das körpernah zu tragende Erzeugnis eine Textile. Vorzugsweise ist die Textile aus Naturfasern, insbesondere aus Baumwollfasern, gebildet.

Es kann ferner vorgesehen sein, dass das körpernah zu tragende Erzeugnis ein Bekleidungsstück ist, das zum Beispiel aus einem textilen Stoff gebildet ist. Insbesondere sind die Bekleidungsstücke solche, die im direkten Hautkontakt getragen werden, zum Beispiel Kapuzenpullover, T-Shirts, Unterwäsche, sportliche Bekleidung, aber auch Tücher und Schals. Ein anderes körpernah zu tragendes Erzeugnis auf textiler Basis ist beispielsweise Bettwäsche.

Gemäß einer weiteren Ausgestaltung der Erfindung ist das körpernah zu tragende Erzeugnis ein Schmuckartikel, zum Beispiel ein Armband, eine Kette oder ein Ring. Die energetisch modifizierte Trägersubstanz des als Schmuckartikel ausgebildeten körpernah zu tragenden Erzeugnisses ist vorzugsweise ein Edelmetall.

Ferner kann das körpernah zu tragende Erzeugnis ein Kosmetik- oder Hautpflegeprodukt sein, zum Beispiel eine Tagescreme, eine Nachtcreme, eine Augencreme, ein Gesichtsreiniger, ein Gesichtstoner, eine Bodylotion, ein Make-up oder ein Körperspray.

## Patentansprüche

1. Körpernah zu tragendes Erzeugnis, **dadurch gekennzeichnet, dass** es mindestens eine energetisch modifizierte Trägersubstanz mit einem durch die magnetischen Momente der Atome und Elementarteilchen definierten elektromagnetischen Zieleigenschaftsspektrum aufweist, wobei die energetisch modifizierte Trägersubstanz aus einer primären Trägersubstanz mit einem durch die magnetischen Momente der Atome und Elementarteilchen der primären Trägersubstanz definierten elektromagnetischen Primäreigenschaftsspektrum gebildet ist, dem zur Ausbildung des Zieleigenschaftsspektrums ein elektromagnetisches Prägeeigenschaftsspektrum aufgeprägt ist, wobei das elektromagnetische Prägeeigenschaftsspektrum durch die magnetischen Momente der Atome und Elementarteilchen einer Prägesubstanz definiert ist.

2. Körpernah zu tragendes Erzeugnis nach Anspruch 1, **dadurch gekennzeichnet, dass** die energetisch modifizierte Trägersubstanz erhältlich ist durch:
- Erfassung des elektromagnetischen Prägeeigenschaftsspektrums der Prägesubstanz bei unterdrücktem Erdmagnetfeld in Form eines Frequenzmusters, und
- Übertragung des erfassten Frequenzmusters auf die Trägersubstanz durch elektromagnetisch induzierte Modifikation der magnetischen Momente der Atome und Elementarteilchen der primären Trägersubstanz bei unterdrücktem Erdmagnetfeld unter Ausbildung der energetisch modifizierten Trägersubstanz mit dem elektromagnetischen Zieleigenschaftsspektrum.

3. Körpernah zu tragendes Erzeugnis nach Anspruch 1 oder 2, **dadurch gekennzeichnet, dass** das die energetisch modifizierte Trägersubstanz enthaltende körpernah zu tragende Erzeugnis eine Textile ist.

4. Körpernah zu tragendes Erzeugnis nach Anspruch 3, **dadurch gekennzeichnet, dass** die Textilie aus Naturfasern gebildet ist.

5. Körpernah zu tragendes Erzeugnis nach Anspruch 4, **dadurch gekennzeichnet, dass** die Textilie aus Baumwollfasern gebildet ist.

6. Körpernah zu tragendes Erzeugnis nach einem der Ansprüche 1 bis 4, **dadurch gekennzeichnet, dass** das die energetisch modifizierte Trägersubstanz enthaltende körpernah zu tragende Erzeugnis ein Bekleidungsstück ist.

7. Körpernah zu tragendes Erzeugnis nach einem der Ansprüche 1 bis 4, **dadurch gekennzeichnet, dass** das die energetisch modifizierte Trägersubstanz enthaltende körpernah zu tragende Erzeugnis Bettwäsche ist.

8. Körpernah zu tragendes Erzeugnis nach Anspruch 1 oder 2, **dadurch gekennzeichnet, dass** das die energetisch modifizierte Trägersubstanz enthaltende körpernah zu tragende Erzeugnis ein Schmuckartikel ist.

9. Körpernah zu tragendes Erzeugnis nach Anspruch 8, **dadurch gekennzeichnet, dass** der Schmuckartikel ein Armband, eine Kette oder ein Ring ist.

10. Körpernah zu tragendes Erzeugnis nach Anspruch 8 oder 9, **dadurch gekennzeichnet, dass** die energetisch modifizierte Trägersubstanz ein Edelmetall ist.

11. Körpernah zu tragendes Erzeugnis nach Anspruch 10, **dadurch gekennzeichnet, dass** die energetisch modifizierte Trägersubstanz Silber oder eine Silberlegierung ist.

12. Körpernah zu tragendes Erzeugnis nach Anspruch 1 oder 2, **dadurch gekennzeichnet, dass** das die energetisch modifizierte Trägersubstanz enthaltende körpernah zu tragende Erzeugnis ein Kosmetik- oder Hautpflegeprodukt ist.

13. Körpernah zu tragendes Erzeugnis nach Anspruch 12, **dadurch gekennzeichnet, dass** das Kosmetik- oder Hautpflegeprodukt eine Tagescreme, eine Nachtcreme, eine Augencreme, ein Gesichtsreiniger, ein Gesichtstoner, eine Bodylotion, ein Make-up oder ein Körperspray ist.

14. Verfahren zur Herstellung einer in körpernah zu tragende Erzeugnisse einzubringenden, energetisch modifizierten Trägersubstanz mit einem durch die magnetischen Momente der Atome und Elementarteilchen definierten elektromagnetischen Zieleigenschaftsspektrum, **dadurch gekennzeichnet, dass** die energetisch modifizierte Trägersubstanz aus einer primären Trägersubstanz mit einem durch die magnetischen Momente der Atome und Elementarteilchen der primären Trägersubstanz definierten elektromagnetischen Primäreigenschaftsspektrum gebildet wird, dem zur Ausbildung des Zieleigenschaftsspektrums ein elektromagnetisches Prägeeigenschaftsspektrum aufgeprägt wird, wobei das elektromagnetische Prägeeigenschaftsspektrum durch die magnetischen Momente der Atome und Elementarteilchen einer Prägesubstanz definiert wird.

15. Verfahren nach Anspruch 14, **dadurch gekennzeichnet, dass**
- das elektromagnetische Prägeeigenschaftsspektrum der Prägesubstanz bei unterdrücktem Erdmagnetfeld in Form eines Frequenzmusters erfasst wird, und
- das erfasste Frequenzmuster auf die Trägersubstanz durch elektromagnetisch induzierte Modifikation der magnetischen Momente der Atome und Elementarteilchen der primären Trägersubstanz bei unterdrücktem Erdmagnetfeld übertragen wird, wobei die energetisch modifizierte Trägersubstanz mit dem elektromagnetischen Zieleigenschaftsspektrum ausgebildet wird.
